(19) Europäisches Patentamt
European Patent Office
Office européen des brevets

(11) Publication number: 0 278 662 A2

(12) **EUROPEAN PATENT APPLICATION**

(21) Application number: 88300880.7

(22) Date of filing: 02.02.88

(51) Int. Cl.[4]: C08K 5/51 , C09J 3/14 , C09J 7/02 , C08J 3/28 , A61L 2/08

(30) Priority: 06.02.87 US 11540

(43) Date of publication of application: 17.08.88 Bulletin 88/33

(84) Designated Contracting States: DE ES FR GB IT NL SE

(71) Applicant: MINNESOTA MINING AND MANUFACTURING COMPANY
3M Center, P.O. Box 33427
St. Paul, Minnesota 55133-3427(US)

(72) Inventor: Kurka, Kurt A. c/o Minnesota Mining and
Manufacturing Co. 2501 Hudson Road P.O. Box 33427
St. Paul Minnesota 55133-3427(US)
Inventor: Cran, Lauren K. c/o Minnesota Mining and
Manufacturing Co. 2501 Hudson Road P.O. Box 33427
St. Paul Minnesota 55133-3427(US)

(74) Representative: Baillie, Iain Cameron et al
c/o Ladas & Parry Isartorplatz 5
D-8000 München 2(DE)

(54) **Organo-phosphorous energy transfer agents.**

(57) The invention concerns ionizing radiation energy transfer agents which are comprised of organo-phosphorous compounds which significantly lower the levels of radiation needed to cure polymers, oligomers, and/or monomers and which impart crosslinking in previously radiation uncrosslinkable polymers.

EP 0 278 662 A2

Xerox Copy Centre

# ORGANO-PHOSPHOROUS ENERGY TRANSFER AGENTS

## Technical Field

This invention relates to ionizing radiation energy transfer agents and particularly to the use of such energy transfer agents in the production of pressure sensitive adhesives and polyolefin articles utilizing lower levels of ionizing radiation.

## Background

The use of radiation to effect the curing of polymers, oligomers, monomers and mixtures thereof is well known in the art. Radiation as used herein refers to high energy radiation and/or the secondary energies resulting from the conversion of electron or other particle energy to neutron or gamma radiation. The curing effect is believed to be largely a free radical mechanism resulting primarily in the crosslinking of the involved compounds with some chain extension also occurring.

When a polymeric compound is subjected to radiation, some degradation of the polymer occurs. Degradation is the breaking of the molecular chains of the polymer. The extent of degradation depends on the level of radiation and the polymeric or molecular structure involved. In certain polymers degradation exceeds crosslinking, resulting in no cure of the compound being evident. In others, even when crosslinking exceeds degradation, sufficient degradation occurs to reduce desired physical properties of the resultant compound. example, paper or polypropylene film. It is often desirable to use an inexpensive backing such as paper for pressure sensitive tape. However, when sufficient radiation energy is applied to cure the adhesive, the backing's tensile strength and flexibility are substantially reduced. Thus, radiation curing typically cannot be employed when these desirable, albeit radiation sensitive, backings are used.

Radiation is also used to sterilize polymeric products and food wrapped in polymeric film. These products and films are also often highly sensitive to radiation degradation. For example, syringes are often made of polypropylene which becomes brittle when sterilized with radiation.

It is also desirable to cure compounds at a lower level of radiation in order to greatly reduce the manufacturing cost. By reducing the level of radiation required, the product can be fed through the radiation device at a much greater rate of speed. This results in significant cost savings for the manufacturer.

Further, certain polymers, such as polypropylene, do not exhibit substantial crosslinking when irradiated. Crosslinking of these materials would be desirable to impart increased strength and heat resistance.

It is, therefore, highly desirable to be able to cure polymers, oligomers and/or monomers with significantly lower levels of radiation and to impart crosslinking in previously radiation uncrosslinkable polymers. The present invention provides ionizing radiation energy transfer agents which can be added to the compounds to be cured, thereby increasing the amount of crosslinking relative to the amount of degradation thus allowing curing to take place at greatly reduced levels of radiation. The compounds of the present invention may also be added to polymers such as polypropylene, to increase the amount of crosslinking.

## Disclosure of the Invention

According to the present invention there is provided:

A novel ionizing radiation curable composition comprising a mixture of:

1) at least one polymer selected from the group consisting of polyolefins, polyaralkenes, diene polymers, halogen substituted diene polymers, halogen substituted polyolfins and acrylic polymers.

2) an effective amount of a pentavalent organophosphorus energy transfer agent represented by the following general formula:

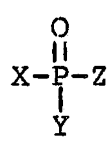

wherein X, Y and Z are independently selected from the group consisting of R, OR', H and OH with X, Y, Z each being different except that when X and Z are both either R or OR', Y is R, OR' H or OH; and when X and Z are both OH, Y is either R or OR'. R and R' are selected from the group consisting of alkyls, aryls, alkyls substituted with a saturated compound (hereinafter saturated substituted alkyls), aryls substituted with a saturated compound (hereinafter saturated substituted aryls), alkyls substituted with an allyl compound (hereinafter allyl substituted alkyls) and aryls substituted with an allyl compound (hereinafter allyl substituted aryls);

The novel ionizing radiation curable compositions of the present invention may be subjected to radiation to cure and become cured compositions of the present invention.

It has been found that the preferred amount of the energy transfer agent ranges from about 0.1 to 25% by weight of the polymer. The composition of the present invention may also contain an effective amount of a crosslinking agent. The preferred amount of the crosslinking agent has been found to be between about 0.1 and 75% by weight of the polymer.

The present invention also contemplates an ionizing radiation curable pressure sensitive adhesive containing the above energy transfer agent and a process for making a cured pressure sensitive adhesive tape from said curable adhesive. Also, a method for sterilizing or crosslinking an article or a package which comprises polypropylene containing the above energy transfer agent is contemplated.

## Detailed Description of the Invention

The novel ionizing radiation curable composition of the present invention comprises a mixture of at least one polymer selected from the group consisting of polyolefins, polyaralkenes, diene polymers, halogen subsituted diene polymers, halogen substituted polyolefins, and acrylic polymers in combination with a pentavalent organophosphorus compound which acts as an energy transfer agent surprisingly allowing curing to be accomplished at significantly lower levels of radiation. Examples of useful polymers include polyisoprene, polybutadiene, polystyrene, polyethylene, polypropylene, polyacrylics, polychloroprene, polyvinylacetate, polyvinylchloride, and copolymers, terpolymers, blockcopolymers and blends thereof.

An energy transfer agent can be described as any compound that facilitates the transfer or migration of excitation energy from the location of absorption along polymer chains to a point of radiation/chemical reaction, without the compound entering into that chemical reaction. This field of energy transfer in a polymer molecule is not an altogether understood area.

Energy transfer has been described as follows: "...the effect of energy absorbed at a given point of the polymer molecule can be felt far from the location where it was absorbed. To describe this delocalization of absorbed energy, the term Energy Transfer is often used. In general terms this concept does not reflect the essence of the process, since not only does transfer of excitation energy take place, but also of electrons, hydrogen atoms, free radicals, etc. The existence of transfer is qualitatively confirmed by the fact that despite random distribution of the absorbed energy, the final radiation --chemical transformations are localized on definite sections of the polymer molecule..." F.A. Makhlis, Radiation Physics and Chemistry of Polymers, page 210, John Wiley & Sons.

It is intended that the term "radiation" as used herein refer to "ionizing radiation", which has been defined as radiation possessing sufficient energy to produce ions or to break chemical bonds, and thus includes "ionizing particle radiation" as well as "ionizing electromagnetic radiation".

"Ionizing electromagnetic irradiation" is produced when a metallic target, such as tungsten, is bombarded with electrons of suitable energy. This energy is conferred to the electrons by potential accelerators of over 0.1 million electron volts (mev.). In addition to irradiation of this type, commonly called X-ray, an ionizing electromagnetic irradiation suitable for the practice of this invention can be obtained by means of a nuclear reactor (pile) or by the use of a natural or synthetic radioactive material, for example, cobalt 60.

The term "ionizing particle radiation" has been used to designate the emission of electrons or highly accelerated nuclear particles such as protons, neutrons, alpha-particles, deuterons, beta-particles, or their analogs, directed in such a way that the particle is projected into the mass to be irradiated. Charged particles can be accelerated by the aid of voltage gradients by such devices as accelerators with resonance chambers, such as Van der Graaff generators, betatrons, synchrotons, cyclotrons, etc. While various types of radiation are suitable for this purpose, such as x-ray and gamma rays, the radiation produced by accelerated high energy electrons has been found to be very conveniently and economically applicable and give very satisfactory results.

The ionizing radiation energy transfer agent of the present invention is a pentavalent organo phosphorous compound represented by the following general formula:

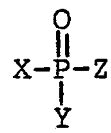

wherein X, Y and Z are independently selected from the group consisting of R, OR′, H or OH each being different, except that when X and Z are both either R or OR′, Y is either R or OR′, H or OH; and when X and Z are both OH, Y is either R or OR′, where R and R′ are selected from the group consisting of alkyls, aryls, saturated substituted alkyls, saturated substituted aryls, allyl substituted alkyls and allyl substituted aryls.

The transfer agent is preferably a triarylphosphate or an alkyl diaryl phosphate, more preferably tricresyl phosphate or 2-ethylhexyldiphenylphosphate. Examples of other suitable energy transfer agents include triphenylphosphate, isodecyl diphenylphosphate, tributylphosphate, bis-2-ethylhexyl-2-ethylhexylphosphate, diisoctyl acid phosphite, triphenyl phosphine oxide, phenyl phosphinic acid.

It has also been found that there is a synergistic effect when a crosslinking agent is also added. Crosslinking agents which may be used include polyfunctional acrylate monomers, polyfunctional acrylate oligomers, polyfunctional allyl compounds, bismaleimides and polymercaptans, a suitable example being trimethylolpropane triacrylate.

The energy transfer agents of the present invention are very useful in curing pressure sensitive adhesive tapes with ionizing radiation. A novel pressure sensitive adhesive tape may be made according to the present invention by coating an adhesive on a substrate, the adhesive comprising an ionizing radiation curable compound comprising a polymer, an energy transfer agent and a tackifying resin, and subjecting the composition to an effective amount of ionizing radiation, typically found to be between 1 and 30 Megarads. 1 Megarad = $10^6$ rads = 10 $kJ.Kg^{-1}$ or $10^8$ ergs. $g^{-1}$ of absorbed energy.

The polymer and the energy transfer agent are selected from those set forth above. The tackifying resin is any suitable tackifying resin known in the art, for example, Piccolyte S115, commercially available from Hercules, Inc. A crosslinking agent may also be added to further reduce the level of radiation required to cure.

The pressure-sensitive adhesive products of this invention are useful as pressure-sensitive adhesive coatings on many substrates, including aluminum foil, steel or other metal panels; non-woven fiber web sheets, plastic substrates such as polyvinyl chloride, polyester and the like; cellulosic substrates such as paper, wood, etc.; woven fabrics such as glass, cotton, nylon, wool and composites of these materials. Some of these backings are subject to degradation when subjected to more than 1 or 2 Megarads. Especially useful are products such as adhesive tapes and the like wherein the adhesive is formed on a thin, flexible backing sheet material. These products may also be used as laminating binders and as supported or unsupported film adhesives. For all these uses and many others not described, the resulting product has exceptional adhesive properties, while capable of being cured at lower levels of radiation.

The energy transfer agent of the present invention also has utility in a process for sterilizing a semi-crystalline polypropylene. When the energy transfer agents of the present invention are added to semi-crystalline polypropylene, degradation of the polypropylene, when subjected to sterilizing amounts of radiation, is reduced.

Comparative Example A

A pressure sensitive adhesive tape was made by mixing the following ingredients:

| | PHR |
|---|---|
| Natural Rubber Millbase (2.7IV) | 100 |
| Tackifying Resin (Piccolyte S115) | 60 |
| $TiO_2$ Pigment | 16 |
| Sandorin Yellow Pigment | 1 |
| Antioxidant (Irganox 1076) | 1 |

The composition was then coated onto a polyester film and dried.

Comparative Example B

A pressure sensitive adhesive tape was prepared according to Example A except 0.5 phr of trimethylolpropane triacrylate, a known crosslinking agent, was also added.

Example 1

A pressure sensitive adhesive tape of the present invention was prepared by adding 10 phr of tricresylphosphate to Example A.

Example 2

A pressure sensitive adhesive of the present invention was prepared by adding 10 phr tricresylphosphate to Example B and coating the adhesive onto a polyester film.

The pressure sensitive adhesive tapes prepared in Examples A, B, 1 and 2 were subjected to radiation levels of 9 and 12 megarads. The following test results were obtained from the radiation cured samples.

| | 9 Megarads | | 12 Megarads | |
|---|---|---|---|---|
| | 130°C Shear(1) | RB Tack(2) | 130°C Shear | RB Tack |
| Example A | 37 min. | 2.3" | 27 min. | 3.6" |
| Example B | 70 min. | 5.6" | 108 min. | 5.7" |
| Example 1 | 147 min. | 0.5" | 303 min. | 0.5" |
| Example 2 | 455 min. | 1.9" | 725 min. | 2.0" |

(1) 1/2 x 1/2 Sample, 500 gm. wt., steel panel

(2) ASTM Test D-3121, Rolling Ball Tack

This data illustrates the improvement in adhesive shear strength with the energy transfer agent TCP over and above that of the crosslinking agent TMPTA. The decrease in tack from the use of TMPTA is offset by the plasticizing action of TCP. Example 2 illustrates the synergism of a crosslinking agent in combination with the phosphate transfer agent.

Comparative Example C

A solution of a styrene/isoprene block copolymer (Kraton 1107, Shell Chemical Co.) containing 1 part of the crosslinking agent bismaleimide M-20 was prepared.

Example 3

A solution according to the present invention was made up by adding 6 parts of tricresylphosphate to the formulation of Comparative Example C.

Comparative Example C and Example 3 were coated on polyester film, dried and subjected to radiation levels of 1, 3 and 5 megarads. The gel-content of each rubber sample was measured by gel-swell techniques, with the following results.

| | Gel Content | | |
|---|---|---|---|
| | 1 Megarad | 3 Megarads | 5 Megarads |
| Comparative Example C | 0% | 0% | 13% |
| Example 3 | 35% | 65% | 80% |

The gel-swell technique measures the amount of gel present in a sample which indicates the amount of cross-linking which has occurred in the sample. This data illustrates the dramatic increase in crosslink density of a composition with the TCP energy transfer agent over the effect of the Bismaleimide crosslinking agent alone.

Examples 4-17

Various energy transfer agents were added to #1 ribbed smoked sheet natural rubber milled on a rubber mill to 2.09 inherent viscosity (I.V.), in varying amounts so as to provide an equivalent level of phosphorous in each. The samples were coated on polyester film, dried and subjected to 4 and 12 Mrads of radiation. The gel contents of each sample was then determined by gel-swell techniques, with the following results.

Comparative Example D is a control with no energy transfer agent contained therein.

| Ex. | | PHR of ETA | 4 Mrads | 12 Mrads |
|---|---|---|---|---|
| D | 2.09 I.V. Natural Rubber | 0 | 56.4 | 85.7 |
| 4 | 2-Ethylhexyl diphenyl phosphate | 5.8 | 79.4 | 90.8 |
| 5 | Tricresyl phosphate | 4.8 | 76.5 | 91.6 |
| 6 | Isodecyldiphenyl phosphate | 6.3 | 74.9 | -- |
| 7 | Triphenyl phosphate | 5.3 | 74.5 | -- |
| 8 | p-tert-Butylphenyldiphenyl phosphate | 6.2 | 73.7 | -- |
| 9 | Phenyl acid phosphate | 3.5 | 67.6 | 88.8 |
| 10 | Diphenyl acid phosphite | 3.0 | 67.2 | 89.5 |
| 11 | Phenylphosphinic Acid | 1.8 | 67.1 | 89.4 |
| 12 | Tridecyl acid phosphate | 6.0 | 66.4 | -- |
| 13 | Tributyl phosphate | 4.3 | 66.0 | 87.6 |
| 14 | Bis(2-ethylhexyl)-2-ethylhexylphosphonate | 6.8 | 65.5 | -- |
| 15 | Diisooctyl acid phosphite | 4.9 | 64.5 | 86.6 |
| 16 | Tri-n-octyl phosphate | 7.0 | 62.9 | -- |
| 17 | Triphenyl phosphine oxide | 3.6 | 61.6 | 86.4 |

Certain Examples were only run at 4 Mrds.

Example 18

An isotactic polypropylene film according to the present invention was made by heating isotactic polypropylene and mixing to a plastic state at 115°C in a sigma blade mixer. Two percent of 2-ethylhexyldiphenyl phosphate (an energy transfer agent) based on the weight of polypropylene was added and thoroughly mixed in. The mixture was allowed to cool and then ground up into chips. This polypropylene/energy transfer agent blend was extruded through a 3/4" extruder at 232°C feeding a die to produce film of 9 mils in thickness.

Comparative Example E

A 9 mil thick isotactic polypropylene film was made according to the procedure of Example 18 except no energy transfer agent was added.

Samples of the films of Example 18 and Comparative Example E were radiated at an acceleration voltage of 175 KV at 1, 3 and 5 megarads under nitrogen. Each side of each sample was separately irradiated to insure complete electron penetration. Each sample as tested with an Instron Tensile tester at a temperature of 160 C. The tensile strength at an elongation of 10% was measured with results, shown as percent of the original unradiated tensile strength, as follows.

| | Megarad Dosage | | | |
|---|---|---|---|---|
| Additive | 0 | 1 | 3 | 5 |
| Comparative Example E | 100% | 58% | 10% | 0% |
| Example 18 | 100% | 183% | 145% | 9% |

The above results show that polypropylene undergoes degradation at all levels of radiation used in this example, but that the addition of an energy transfer agent reverses this effect in favor of crosslinking through 3 megarads of radiation, providing an improvement in properties while subjected to a level of radiation sufficient to provide sterilization.

Comparative Example F

10 grams of CMD 6700 (an acrylated urethane polymer with a functionality of 2.2 and a molecular weight of 1527, sold commercially by Interez Inc.) was added to 100 grams of natural rubber that had been milled to a 2.2 I.V. and dissolved in toluene. This sample was coated onto a polyester film carrier, dried in an oven and cut into samples that were individually radiated at either 1, 2, 3, 4 or 5 megarads.

Example 19

A composition of the present invention was made as in Comparative Example F above, with the exception that 5 grams of tricresyl phosphate were added and mixed prior to coating and drying. Comparative Example F and Example 19 were radiated at 1, 2, 3, 4 or 5 megarads. The gel content of each radiated sample of these two compositions were determined by sol gel measurements from a 72 hour toluene solvent soak with the following results.

% GEL CONTENT

| | Megarads | | | | | |
|---|---|---|---|---|---|---|
| | 0 | 1 | 2 | 3 | 4 | 5 |
| Comparative Example F | 0 | 1.7 | 39.2 | 57.4 | 67.4 | 74.8 |
| Comparative Example 19 | 0 | 26.3 | 59.2 | 72.5 | 79.8 | 83.1 |

Example 20-21, Comparative Examples G, H

An adhesive base was made by mixing 100 parts of masticated natural rubber with an inherent viscosity of 2.1, 70 parts of Escorez 1310 tackifier and 1 part of Irganox 1010 antioxidant, with and without the energy transfer agent, tricresyl phosphate and the cross-linking agent, Bismaleimide M-20. (Comparative Examples G, H) Bismaleimide M-20 is available commercially from MTC America, Inc., 200 Park Avenue, New York, NY. Each formulation was coated from toluene, dried and radiation cured at 1, 2, and 3 megarads in an inert atmosphere.

A room temperature shear measurement (PSTC Method 7, 1/2" × 1/2" test sample, 1000 gram weight) was run on the Examples with the results below.

| Example | Additives to Rubber Resin Adhesive Base | Adhesion to Steel RT Shearholding in Minutes 1 Megarad | 2 Megarad | 3 Megarads |
|---|---|---|---|---|
| G | None | <2 | 26 | 33 |
| H | 1 phr Bismaleimide M-20 | <2 | 49 | 59 |
| 21 | 5 phr Tricresyl-phosphate | <2 | 55 | 60 |
| 22 | 1 phr Bismaleimide M-20 5 phr Tricresyl-phosphate | <2 | 100 | 108 |

The 180° peel adhesion to a glass surface at 90"/minute of Examples G, H 21 and 22 were all in the range of 60±5 oz/in. originally, and were largly unaffected by the radiation cure, retaining their original values in the range of 60±5 oz/in.

**Claims**

1. A ionizing radiation cured composition characterized by the feature that said composition initially comprises a mixture of

1) at least one polymer selected from the group consisting of polyolefins, polyaralkenes, diene polymers, halogen substituted diene polymers, acrylic polymers, and halogen substituted polyolefins; and

2) an effective amount of a pentavalent organophosphorus energy transfer agent represented by the following general formula:

```
   O
   ||
X-P-Z
   |
   Y
```

wherein X, Y and Z are independently selected from the group consisting of R, OR', H or OH, each being different with the exception; when X and Z are both either R or OR', Y is R, OR' H or OH; when X and Z are both OH, Y is either R or OR, and wherein R and R' are selected from the group consisting of alkyls, aryls, saturated substituted alkyls, saturated substituted aryls, allyl substituted alkyls and allyl substituted aryls.

2. The composition of claim 1 further including an effective amount of a crosslinking agent, said crosslinking agent being selected from the group consisting of polyfunctional acrylate monomers, polyfunctional acrylate oligomers, polyfunctional allyl compounds, bismalemides and polymercaptans.

3. The composition of claim 1 wherein said at least one polymer is selected from the group consisting of polyisoprene, polybutadiene, polystyrene, polyethylene, polypropylene, polyacrylics, polychloropene, polyvinylchloride, polyvinylacetate and copolymers, terpolymers, block copolymers, and blends thereof.

4. A pressure sensitive adhesive tape comprising a cured adhesive on a substrate, characterized by the feature that said adhesive comprises the ionizing radiation cured composition of claim 1 initially comprising:

1) at least one polymer selected from the group consisting of polyolefins, polyaralkenes, diene polymers, halogen substituted diene polymers, acrylic polymers and halogen substituted polyolefins; and

2) an effective amount of a pentavalent organophosphorus energy transfer agent compound represented by the following general formula:

```
   O

X-P-Z

   Y
```

wherein X, Y and Z are independently selected from the group consisting of R, OR', H or OH, each being different with the exception that; when X and Z are both either R or OR', Y is R, OR' H or OH; when X and Z are both OH, Y is either R or OR, and wherein R and R' are selected from the group consisting of alkyls, aryls, saturated substituted alkyls, saturated substituted aryls, allyl substituted alkyls and allyl substituted aryls; and

3) a tackifying resin.

5. The tape of claim 4 wherein said composition further includes an effective amount of a crosslinking agent being, said crosslinking additive selected from the group consisting of polyfunctional acrylate monomers, polyfunctional acrylate oligomers, polyfunctional allyl compounds, bismaleimides and polymercaptans.

6. The product of claim 1 or claim 4 wherein said energy transfer agent is present in an amount in the range of about 0.1 to about 25 percent by weight of said polymer.

7. The product of claim 2 or claim 5 wherein said crosslinking additive is present in an amount in the range of about 0.1 to 75 percent by weight of the polymer.

8. The adhesive of claim 4 wherein said at least one polymer is selected from the group consisting of polyisoprene, polybutadiene, polystyrene, polyethylene polypropylene, polyacrylics, polychloropene, polyvinylacetate, copolymers, terpolymers, block copolymers and blends thereof.

9. The product of claim 1 or claim 4 wherein said organophosphorous compound is selected from the group consisting of triarylphosphate, alkydiarylphosphate, triphenylphosphate, isodecyl diphenylphosphate, tributylphosphate, bis-2-ethylhexyl-2-ethylhexyl phosphate, diisoctyl acid phosphite, triphenyl phosphine oxide, and phenyl phosphinic acid.

10. The tape of claim 4 wherein said substrate is subject to degradation when subjected to more than .1 total Megarads.

11. The tape of claim 10 wherein said substrate is selected from the group consisting of cellulose materials, polypropylene, polyisobutylene, polytetrafluoroethylene, polypropylene oxide.

12. A process for preparing the ionizing radiation cured pressure sensitive adhesive tape of claim 4 comprising a) applying to a substrate a composition, characterized by the feature that said composition initially comprises:

1) at least one polymer selected from the group consisting of polyolefins, polyaralkenes, diene polymers, halogen substituted diene polymers, acrylic polymers and halogen substituted polyolefins; and

2) an effective amount of a pentavalent organophosphorus compound represented by the following general formula:

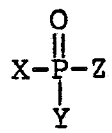

wherein X, Y and Z are independently selected from the group consisting of R, OR′, H or OH, each being different with the exception; when X and Z are both either R or OR′, Y is R, OR′, H or OH; when X and Z are both OH, Y is either R or OR, and wherein R and R′ are selected from the group consisting of alkyls, aryls, saturated substituted alkyls saturated substituted aryls, allyl substituted alkyls and allyl substituted aryls; and

3) a tackifying resin

b) subjecting said composition to an effective amount ionizing radiation to cure said composition.

13. A process for sterilizing a semi-crystalline polypropylene, characterized by the feature that said process comprises:

1) subjecting to a sterilizing amount of high energy radiation, a semi-crystalline polymer having a crystalline content of from 5 to 95% and an energy transfer agent represented by the following general formula:

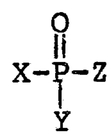

wherein X, Y and Z are independently selected from the group consisting of R, OR′, H or OH each being different with the exception that when X and Z are both either R or OR′, Y is R, OR′, H or OH; when X and Z are both OH, Y is either R or OR′; and wherein R and R′ are selected from the group consisting of alkyls, aryls, saturated substituted alkyls saturated substituted aryls, allyl substituted alkyls and allyl substituted aryls.